Europäisches Patentamt

European Patent Office

Office européen des brevets

(11) Numéro de publication: **0 179 675**
**B1**

(12) **FASCICULE DE BREVET EUROPÉEN**

(45) Date de publication du fascicule du brevet:
06.12.89

(21) Numéro de dépôt: 85401623.5

(22) Date de dépôt: 09.08.85

(51) Int. Cl.⁴: **A 61 K 7/04,** A 61 K 7/06,
A 61 K 31/23

(54) Composition lipidique favorisant la pousse des phanères et produits cosmétiques en renfermant.

(30) Priorité: 22.08.84 FR 8413057

(43) Date de publication de la demande:
30.04.86 Bulletin 86/18

(45) Mention de la délivrance du brevet:
06.12.89 Bulletin 89/49

(84) Etats contractants désignés:
BE CH LI LU

(56) Documents cité:
FR-A-2 097 036
FR-A-2 167 407
FR-A-2 213 052
FR-A-2 255 055
FR-A-2 467 598
US-A-2 954 325
US-A-4 286 609

(73) Titulaire: **Mollet, Marc, 38, rue des Chevaliers de St Jean, F-91100 Corbeil Essonnes (FR)**
Titulaire: **Galli, Jeanne, 35 rue Mirabeau, F-75016 Paris (FR)**
Titulaire: **Galli, André, 35 rue Mirabeau, F-75016 Paris (FR)**
Titulaire: **Leluc, Robert, 49 rue Jeanne d'Arc, F-94160 St. Mande (FR)**

(72) Inventeur: **Mollet, Marc, 38, rue des Chevaliers de St Jean, F-91100 Corbeil Essonnes (FR)**
Inventeur: **Galli, Jeanne, 35 rue Mirabeau, F-75016 Paris (FR)**
Inventeur: **Galli, André, 35 rue Mirabeau, F-75016 Paris (FR)**
Inventeur: **Leluc, Robert, 49 rue Jeanne d'Arc, F-94160 St. Mande (FR)**

(74) Mandataire: **Levy, David, c/o S.A. Fedit- Loriot 38, avenue Hoche, F-75008 Paris (FR)**

LIBER, STOCKHOLM 1989

## Description

La présente invention concerne les produits favorisant la pousse des phanères et plus particulièrement les cheveux, les cils et les ongles.

Depuis longtemps, on a recherché à favoriser la pousse des phanères, soit dans un but esthétique, soit pour pallier des déficiences organiques ou des agressions externes, notamment dues à l'environnement et plus particulièrement aux vapeurs de substances diverses qui imprègnent l'atmosphère des villes ou des lieux de travail, ainsi que certains produits détersifs ou odoriférants utilisés pour l'hygiène de la chevelure.

On a proposé dans ce sens des produits très divers, certains à base de graisses animales, telles le chignon de cheval, ou encore des sécrétions animales comme la gelée royale d'abeille.

On a également proposé diverses vitamines ou des extraits naturels tels que l'écorce de quinquina, le henné et bien d'autres.

Toutes les substances préconisées jusqu'ici, et plus particulièrement celles à base d'extraits animaux ou végétaux donnaient des résultats variables suivant les individus, mais également suivant la fabrication dont la composition varie dans le temps selon l'origine des matières premières.

Une première amélioration a été obtenue par élimination des matières cellulaires et proté i ques des graisses animales, par exemple suivant le procédé décrit dans le brevet français n° 1.442.280.

On évitait ainsi certains phénomènes allergiques et on obtenait une meilleure pénétration des produits actifs mais on a constaté cependant des différences d'efficacité jusqu'ici peu expliquées.

Ainsi le brevet des Etats-Unis d'Amérique US-A-4 286 609 a proposé un mélange d'huile de soja, d'huile de vison, d'huile de sésame, d'huile d'olive, d'huile de lin et d'huile de coton, pour le traitement des ongles et du cuticule.

Le brevet français FR-A-2 097 036 préconise des compositions comprenant de l'huile de soja, de germes de maïs, de tournesol, de germes de blé, et de pépins de raisin pour favoriser le renouvellement des tissus cutanés.

Le brevet français FR-A-2 167 407 revendique un produit capillaire pour la repousse et les soins du cuir chevelu constitué par un mélange de deux huiles végétales et de deux graisses animales, plus particulièrement un mélange d'huile d'amandes douces, d'huile de ricin, de moelle de boeuf et de graisse de vison.

Le brevet français FR-A-2 213 052 propose un produit revitalisant des ongles humains, et pour leur nettoyage à base de graisse de vison.

Toutes ces compositions donnent des résultats généralement peu prononcés et surtout très variables; sans qu'on ait réussi à découvrir la source de ces variations d'activité.

Des recherches prolongées effectuées par les inventeurs ont montré l'intérêt de certaines préparations lipidiques et ont permis de préciser que la teneur en certains acides gras en conditionnait l'activité.

Les inventeurs ont pu alors découvrir que l'activité des substances lipidiques pour la pousse des phanères était liée à la présence de certains triglycérides dans des proportions bien déterminées.

L'invention a ainsi pour but des compositions lipidiques favorisant la pousse des phanères, d'une efficacité régulière supérieure à celle des produits jusqu'ici connus.

L'invention a pour objet une composition lipidique favorisant la pousse des phanères, fluide à la température ambiante normale, renfermant des triglycérides d'acides gras à 18 atomes de carbone insaturés à trois doubles liaisons et des triglycérides d'acides gras à 16 atomes de carbone saturés et non saturés, composition caractérisée en ce que ces triglycérides y sont présents conjointement dans la proportion de 5 à 70 % de triglycérides à 18 atomes de carbone insaturés à trois doubles liaisons et 95 à 30 % de triglycérides à 16 atomes de carbone dont au moins un tiers sont insaturés à une double liaison.

Suivant un mode de réalisation pratique, une composition lipidique suivant l'invention est constituée à plus de 99 % par un mélange de triglycérides dont plus de 97 % sont des dérivés d'acides gras ayant de 14 à 18 atomes de carbone.

Une telle composition peut renfermer, au plus, environ 1 % d'eau ou d'insaponifiables à plus de 95 % de cholestérol.

Les compositions lipidiques suivant l'invention ont une viscosité d'au plus 8 Pascal/seconde à 20°C et fréquemment voisine de 1,5 Pascal/seconde. Cette fluidité qui différencie les compositions suivant l'invention des substances graisseuses ou huileuses, plus visqueuses, jusqu'ici proposées, constitue également un facteur d'efficacité du fait des facilités accrues d'application et de pénétration dans la peau.

Suivant un mode de réalisation pratique, au moins une partie des triglycérides d'acides gras en $C_{16}$ insaturés provient d'extraits d'au moins une substance animale telle que l'huile de vison et l'huile de pied de boeuf.

On peut également les extraire de graisses animales telles le chignon de cheval. De même, au moins une partie des triglycérides d'acide gras en $C_{18}$ à triple insaturation des compositions suivant l'invention peut provenir d'extraits végétaux, et notamment d'au moins une des huiles de soja, de colza, de noix et de pépins de framboise.

Ainsi on peut réaliser une composition suivant l'invention aussi bien par mélange des triglycérides isolés, quelqu'en soit leur provenance naturelle ou synthétique, que par des extraits de corps gras animaux et/ou végétaux, ou le mélange de produits naturels, isolés et/ou synthétiques.

Dans un mode de réalisation pratique particulièrement intéressant, au moins une partie des triglycérides d'acides gras en $C_{16}$ et $C_{18}$ provient d'un mélange purifié de 85 % d'huile de vison et 15 % d'huile de pépins de framboise.

L'invention a également pour objet des produits cosmétiques, notamment des shampooings, des lotions pour les cheveux, des huiles pour les cils, des crèmes pour les ongles, des vernis à ongles, des dissolvants de vernis à ongles, renfermant de 1 à 40 % de composition lipidique suivant l'invention.

L'invention sera mieux comprise à la lecture de la description détaillée qui va suivre de plusieurs exemples non limitatifs de modes de réalisation suivant l'invention.

Lorsque l'on part de matières premières naturelles animales ou végétales, il convient d'isoler les acides gras en $C_{16}$

saturés et en $C_{16}$ à une double liaison ainsi que les acides gras en $C_{18}$, plus particulièrement à trois doubles liaisons (acide linolénique).

Entre autres procédés connus d'extraction des huiles ou graisses naturelles, on peut les soumettre, séparément ou en mélange, à une hydrolyse, de préférence par de la vapeur d'eau à l'abri de l'air. On élimine ensuite la glycérine par lavage et on distille sous vide avec des colonnes à haut pouvoir séparateur.

On recueille séparément l'extrait désiré : $C_{16}$ : 0, $C_{16}$ : 1, $C_{18}$ : 3 et éventuellement : $C_{18}$ : 0, $C_{18}$ : 1, $C_{18}$ : 2.

La séparation entre les acides gras d'un même nombre d'atomes de carbone présente souvent quelques difficultés techniques. Il est donc particulièrement intéressant de choisir la matière première en conséquence.

Enfin on estérifie les acides gras produits obtenus par de la glycérine. Cette estérification peut se faire avec un mélange stoechiométrique, ou avec un léger excès d'acide gras, dans un réacteur sous vide partiel ou gaz inerte, tel que l'azote à une température de l'ordre de 150 à 200°C avec élimination de l'eau formée, afin de déplacer l'équilibre réactionnel.

On peut bien entendu utiliser des catalyseurs alcalins ou acides classiques afin d'obtenir le maximum de triglycérides. On opèrera dans du matériel en acier inoxydable pour éviter la formation de savons métalliques et la pollution par des métaux lourds.

Si on utilise comme matière première une graisse naturelle telle que le tissu adipeux de cheval, on commencera par la traiter de façon classique pour éliminer l'eau et les matières protéiques. On éliminera ensuite les glycérides saturés, par frigellisation ou winterisation, après fusion à l'air libre et stabilisation prolongée à environ 30°C avant séparation de l'insoluble par décantation.

Une nouvelle stabilisation à environ 20°C suivie d'une filtration ou d'une centrifugation permet d'obtenir une huile fluide, limpide, sans cristaux apparents que l'on traite par frigellisation ou winterisation (fractionnement à sec) sans aucune addition d'autre substance, notamment de conservateur.

La frigellisation peut consister à amener progressivement l'huile à une température de 15° environ par abaissement progressif d'environ 1°C par heure. Après stabilisation d'environ 12 heures à l5°C on filtre pour éliminer l'ensemble constitué par des cristaux de triglycérides indésirables.

La composition lipidique suivant l'invention sera obtenue, soit par un mélange des diverses fractions obtenues suivant le procédé précité, ou tout autre procédé connu, de façon à obtenir les proportions correspondant à une composition suivant l'invention.

On peut également ajouter un triglycéride isolé à une huile naturelle en proportion calculée pour obtenir une composition présentant les proportions caractéristiques de l'invention en acide gras, en $C_{16}$ et $C_{18}$.

On peut extraire les substances actives, comme indiqué ci-dessus ou de façon analogue, de corps gras dont l'analyse a montré que leur mélange dans des proportions déterminées permettait d'obtenir la composition suivant l'invention.

C'est ainsi qu'on pourra utiliser un mélange volume/volume de 85 % d'huile de vison et 15 % d'huile de pépins de framboise.

En effet, les huiles de vison renferment en moyenne

21 % d'acide gras en $C_{16}$ 0
16 % d'acide gras en $C_{16}$ 1
0,8 % d'acide gras en $C_{18}$ 3

L'huile de pépins de framboise renferme en moyenne

2,7 % d'acide gras en $C_{16}$ et
32,6 % d'acide gras en $C_{18}$ : 3.

Dans un mélange à 85 % d'huile de vison et 95 % d'huile de pépins de framboise, on obtient une composition renfermant environ :

15 % d'acide gras en $C_{18}$ : 3
85 % d'acide gras en $C_{16}$ dont
57,5 % en $C_{16}$ : 0 et
43,5 % en $C_{16}$ : 1

Parmi les autres corps gras naturels, l'huile de pied de boeuf renferme généralement de :

15 à 18 % d'acide gras en C 16 : 0
8 à 10 % d'acide gras en C 16 : 1 et
0,5 à 1 % d'acide gras en C 18 : 3.

L'huile de noix renferme de :

7,5 à 8,9 % d'acide gras en C 16 : 0
0 à 0,2 % d'acide gras en C 16 : 1, et
10,8 à 18,6 % d'acide gras en C 18 : 3.

L'huile de soja renferme en moyenne :

10,6 % d'acide gras en C 16 : 0
0,3 % d'acide gras en C 16 : 1
7,4 % d'acide gras en C 18 : 3

On voit que l'on dispose d'une large gamme de possibilités de mélange de substances naturelles permettant d'obtenir des compositions suivant l'invention.

L'analyse d'une composition suivant l'invention, obtenue à partir de mélange de corps gras naturels purifiés, révèle une teneur à 99,8 % de triglycérides dont 98,7 % dérivés d'acide gras de $C_{14}$ à $C_{18}$, dont 35 % d'acide gras en $C_{16}$, avec un rapport de 0,36 entre les acides gras en $C_{16}$ insaturés et $C_{16}$ saturés, et 5,5 % en acide linolénique en $C_{18}$ : 3.

La composition renferme également 0,2 : d'insaponifiable dont les stérols sont constitués à 98,3 % de cholestérol.

Quel que soit le mode de préparation de la composition suivant l'invention, on assure sa conservation par addition d'un anti-oxydant classique tel que l'acide nordihydroguaiarétique (NDGA) à la concentration classique de 1 pour 10 000.

On vérifie les propriétés des compositions suivant l'invention par des essais comparatifs sur l'animal.

A cet effet, on constitue des lots de 10 lapins mâles et femelles fauves de Bourgogne, de poids voisins, que l'on épile sur la région dorsale par action d'une solution de monosulfure de sodium à 20 %.

On traite les lapins chaque jour pendant deux semaines par application sur la partie droite épilée de 0,5 ml de la composition lipidique suivant l'invention.

Dans une première série d'essais, on applique sur la partie gauche épilée une quantité égale à 0,5 ml d'huile d'olive Codex.

Au bout de deux semaines, on constate une repousse plus importante en densité et longueur du pelage sur la partie droite ayant reçu la composition lipidique suivant l'invention.

Dans une deuxième série d'essais sur un lot analogue de lapins, on applique sur la partie droite épilée 0,5 ml de la composition lipidique suivant l'invention, et sur la partie gauche épilée de la graisse semi solide extraite du chignon de cheval.

On constate au bout de deux semaines une repousse plus importante mais plus régulière du pelage sur la partie droite traitée avec la composition lipidique suivant l'invention.

La régularité de l'action se constate également d'un lapin à l'autre.

Dans une troisième série d'essais sur un lot analogue de lapins, on applique sur la partie droite épilée 0,5 ml de la composition lipidique suivant l'invention et sur la moitié gauche une préparation contenant des principes réputés actifs, dans la proportion de 20 ml de liquide amniotique, 0,1 g de panthénol dans 100 ml d'excipient hydroalcoolique q.s.p.

Au bout de deux semaines, on constate une repousse d'au moins 10 mm sur la partie droite traitée avec la composition suivant l'invention, alors que sur la partie gauche traitée avec la préparation classique, on ne constate qu'une repousse inférieure à 5 mm.

Des études histochimiques et histoenzymatiques ont été réalisées pour déterminer l'activité biologique, relative à la croissance des phanères, de la composition lipidique suivant l'invention.

Comme il est classique de faire en cosmétologie, lorsque l'on veut apprécier au niveau du derme une action trophique, toutes ces études ont été réalisées sur des lapins épilés par action du monosulfure de sodium puis frictionnés au moyen de la composition lipidique.

Du point de vue histochimique, on constate une accumulation très importante de substrats biochimiques intervenant dans le métabolisme des poils : mucopolysaccharides nécessaires à la formation bulbe pileux, polysaccharides neutres et de glycolipides, attestant des "gaines" en voie de développement, groupements sulfhydriles témoins d'une production d'acides aminés soufrés dont la cystéine, nécessaires au développment normal des poils.

Les mucopolysaccharides sont mis en évidence par la réaction classique au bleu de toluidine/PAS (acide périodique SCHIFF), les polysaccharides neutres et les glycolipides uniquement par la méthode du PAS, et les groupements sulfhydriques (- SH) selon la technique de FEDERIC et CHEVREMON.

Du point de vue histoenzymatique, les réactions pratiquées montrent une très nette augmentation de l'activité de certaines enzymes spécifiques de la croissance des phanères.

Ces études histoenzymatiques sont réalisées au niveau des glandes sébacées, tissu de formation du poil, au niveau du bulbe pileux, et au niveau du poil proprement dit (gaines).

On constate :

- au niveau des glandes sébacées, avec la glucose 6 phosphatase déshydrogénase (G6 PD), l'apparition de glandes sébacées de volume et d'activités sécrétoires (sébum) plus importants. Ce qui est en liaison avec le métabolisme oxydatif du glucose et la formation de NADPH nécessaire aux biosynthèses des corps gras lubréfiant le poil.
- Au niveau du bulbe pileux et au niveau des gaines du poil :
- la présence de deux enzymes responsables de la néoglycogénèse (glucose 6 phosphatase ou G6P A et lacticodéshydrogénase (LDH) dont l'activité indique qu'il s'agit de poils en voie de constitution (phase anagène).
- la présence de deux phosphatases : la phosphatase alcaline (Alpha GT A) et la 5' nucléotidase ou adénosine 5' monophosphatase (AMP A). L'activité phosphatasique alcaline est liée à la formation de protéines fibreuses (kératine) et stimule le métabolisme des glycérophosphatides et des phospholipides en général, ces phosphatides intervenant dans le métabolisme des membranes cellulaires dont une phospholipase A2 en libère un acide gras essentiel, l'acide arachidonique (acide gras insaturé à 4 doubles liaisons en C20). La 5' nucléotidase participe au métabolisme des nucléotides nécessaires à la croissance des poils dans toutes les espèces animales.
— Enfin, la présence de malicodéshydrogénase ou MD qui intervient dans les phénomènes respiratoires au niveau des cellules vivantes.

Pour essayer la composition lipidique chez l'homme, on administre le produit sous forme d'une solution à 40 pour cent dans un hydrocarbure (en l'espèce le dodécane) suivant un protocole bien codifié :

La dose journalière est représentée par une ampoule de 5 ml de solution à 50 pour cent, mise au contact du cuir chevelu en opérant raie par raie et en massant si nécessaire pour faire pénétrer le produit. Au gré du patient 4 heures au moins après l'application, on pratique un rinçage a l'eau tiède ou un shampooing doux. La durée du traitement est de 4 mois.

Le traitement est appliqué à 20 sujets :

— 10 hommes avec creusement des golfes temporaux et/ou tonsures au niveau du vertex,
— 10 femmes avec alopécies diffuses ou très sévères.

Le produit est bien toléré.
L'arrêt de la chute - apprécieé au test de la traction est net dans tous les cas.
La réponse, appréciée par observation du cuir chevelu et photographie avant et après traitement, est nette dans 60 % des cas, appréciable dans les autres.
La séborrhée, lorsqu'elle existait, est améliorée dans la moitié des cas. L'état pelliculaire a régressé dans 3 cas sur sept.
On réalise des produits cosmétiques suivant l'invention par incorporation d'une composition lipidique suivant l'invention dans des formules classiques et suivant le mode opératoire habituel.
L'incorporation des compositions lipidiques suivant l'invention s'effectue de la même façon que pour les incorporations de corps gras divers dans des produits cosmétiques courants, de préférence la place de ceux-ci,
Les exemples de formules suivants de produits cosmétiques orienteront le technicien:

**Shampooing -**

| | | |
|---|---|---|
| Composition lipidique | 1 | gramme |
| Pendécamaïne | 10 | grammes |
| Acide citrique q.s.p. PH 7.5 | | |
| Sulfate de sodium | 0,5 | gramme |
| Para hydroxy benzoate de méthyle | 0,15 | gramme |
| Eau déminéralisée q.s.p. | 100 | grammes |

**Lotion -**

| | | |
|---|---|---|
| Composition lipidique | 1 | gramme |
| Acide citrique | 0,16 | gramme |
| Alcool à 96° | 25,5 | grammes |
| Para hydroxy benzoate de méthyle | 0,15 | gramme |
| Dodécane | 6,5 | grammes |
| Eau q.s.p. | 100 | grammes. |

**Crème pour les ongles.**

| | | |
|---|---|---|
| Composition lipidique | 20 | grammes |
| Stéarate de triéthanolamine | 11 | " |
| Blanc de baleine | 7,4 | " |
| Stéarate de glycol | 3,7 | " |
| Span 40 (ester d'alcools hexyliques) | 2,2 | " |
| Tween 40 (condensats de Span et d'oxyde d'éthylène) | 2,2 | " |
| BHT (Butyl-hydroxy-toluène) | 0,12 | " |
| Eau q.s.p. | 100 | grammes |

**Vernis à ongles (base)**

| Composition lipidique | 1 | gramme |
|---|---|---|
| nitrocellulose | 5 | " |
| acétate d'amyle | 50 | " |
| acétone | 4 | " |
| alcool amylique | 40 | " |

**Préparation pour les cils**

| Composition lipidique | 1 | gramme, |
|---|---|---|
| huile de ricin | 99 | grammes |

**Dissolvant**

| Composition lipidique | 1 | gramme |
|---|---|---|
| Ethylène diglycol | 8 | " |
| Alcool absolu | 20 | " |
| Méthylal q.s.p. | 100 | " |

**Ampoules pour traitement capillaire -**

| Composition lipidique | 20 à 40 grammes |
|---|---|
| Hydrocarbure saturé (dodécane, hexane) q.s.p. 100 ml en ampoules de 5 ou 10 ml. | |

**Revendications**

1. Composition lipidique favorisant la pousse des phanères, fluide à la température ambiante normale, renfermant des triglycérides d'acides gras à 18 atomes de carbone insaturés à trois doubles liaisons et des triglycérides d'acides gras à 16 atomes de carbone saturés et non saturés, composition caractérisée en ce que ces triglycérides y sont présents conjointement dans la proportion de 5 à 70 % de triglycérides à 18 atomes de carbone insaturés à trois doubles liaisons et 95 à 30 % de triglycérides à 16 atomes de carbone, dont au moins un tiers sont insaturés à une double liaison.

2. Composition lipidique suivant la revendication 1, caractérisée en ce qu'elle est constituée à plus de 99 % par un mélange de triglycérides dont plus de 97 % sont des dérivés d'acides gras ayant de 14 à 18 atomes de carbone.

3. Composition lipidique suivant la revendication 2, caractérisée en ce qu'elle renferme, au plus, 1 % d'eau ou d'insaponifiables à plus de 95 % de cholestérol.

4. Composition lipidique suivant l'une des revendications 1 à 3, caractérisée en ce qu'elle a une viscosité d'au plus 8 Pascal/seconde à 20°C.

5. Composition lipidique suivant l'une des revendications 1 à 4, caractérisée en ce qu'au moins une partie des triglycérides d'acide gras en C 16 insaturés provient d'extraits d'au moins une des huiles de vison et de pied de boeuf.

6. Composition lipidique suivant l'une des revendications 1 à 5, caractérisée en ce qu'au moins une partie des triglycérides d'acide gras en C 18 à triple insaturation provient d'extraits d'au moins une des huiles de soja, de colza, de noix et de pépins de framboise.

7. Composition lipidique, suivant l'une des revendications 5 et 6 caractérisée en ce qu'elle est constituée, au moins partiellement, par des extraits de corps gras animaux et/ou végétaux.

8. Composition lipidique suivant les revendications 5 à 7, caractérisée en ce qu'au moins une partie des triglycérides d'acides gras en C 16 et C 18 provient d'un mélange purifié de 85 d'huile de vison et 15 % d'huile de pépins de framboise.

9. Produit cosmétique, notamment shampooing, lotion pour les cheveux, huile pour les cils, crème pour les ongles, vernis à ongles, dissolvant de vernis à ongles, caractérisé en ce qu'il renferme de 1 à 40 % de composition lipidique suivant l'une des revendications 1 à 8.

**Patentansprüche**

1. Lipidzusammensetzung, die das Wachsen von Hautanhangsgebilden begünstigt, die bei normaler Umgebungstemperatur flüssig ist, die Triglyceride von ungesättigten Fettsäuren mit 18 Kohlenstoffatomen und 3 Doppelbindungen sowie Triglyceride von gesättigten und nicht gesättigten Fettsäuren mit 16 Kohlenstoffatomen enthält, dadurch gekennzeichnet, daß die Triglyceride zusammen in einem Verhältnis vorhanden sind

   – von 5 % bis 70 % der ungesättigten Triglyceride mit 18 Kohlenstoffatomen und 3 Doppelbindungen und
   – 95 % bis 30 % der Triglyceride mit 16 Kohlenstoffatomen, von denen wenigstens ein Drittel ungesättigt mit einer Doppelbindung sind.

2. Lipidzusammensetzung gemäß Anspruch 1, dadurch gekennzeichnet, daß die Zusammensetzung zu mehr als 99 % aus einer Mischung von Triglyceriden, von denen mehr als 97 % Fettsäurederivate sind, die 14 bis 18 Kohlenstoffatome aufweisen, zusammengesetzt ist.

3. Lipidzusammensetzung gemäß Anspruch 2, dadurch gekennzeichnet, daß die Zusammensetzung höchstens 1 % Wasser oder Unverseifbares mit mehr als 95 % an Cholesterin enthält.

4. Lipidzusammensetzung gemäß einem der Ansprüche 1 bis 3, dadurch gekennzeichnet, daß die Zusammensetzung eine Viscosität von höchstens 8 Pascal/Sekunden bei 20°C aufweist.

5. Lipidzusammensetzung gemäß einem der Ansprüche 1 bis 4, dadurch gekennzeichnet, daß die Zusammensetzung zu wenigstens einem Teil der Triglyceride der C 16 ungesättigten Fettsäuren aus Extrakten von wenigstens Nerzöl und Ochsenfuß stammen.

6. Lipidzusammensetzung gemäß einem der Ansprüche 1 bis 5, dadurch gekennzeichnet, daß die Zusammensetzung zu wenigstens einem Teil der Triglyceride der C 18 ungesättigten Fettsäuren aus Extrakten von wenigstens Sojaöl, Rapsöl, Nußöl und Himbeerkernöl stammt.

7. Lipidzusammensetzung gemäß den Ansprüchen 5 und 6, dadurch gekennzeichnet, daß die Zusammensetzung zumindest teilweise aus Extrakten von tierischen und/oder pflanzlichen Fettstoffen zusammengesetzt ist.

8. Lipidzusammensetzung gemäß den Ansprüchen 5 bis 7, dadurch gekennzeichnet, daß die Zusammensetzung zu wenigstens einem Teil der Triglyceride der C 16 und C 18 Fettsäuren aus einer gereinigten Mischung von 85 % Nerzöl und 15 5 Himbeerkernöl stammt.

9. Kosmetisches Produkt, insbesondere Shampoo, Haarlotion, Wimpernöl, Nagelcreme, Nagellack, Nagellackentferner, gekennzeichnet durch, einen Gehalt von 1 % bis 40 % der Lipidzusammensetzung gemäß einem der Ansprüche 1 bis 8.

**Claims**

1. A lipidic composition for promoting the development of superficial body growths liquid at normal room temperature, comprising unsaturated triglycerides of fatty acids with 18 carbon atoms with three double bonds and saturated and non-saturated triglycerides of fatty acids with 16 carbon atoms, the composition being characterised in that these triglycerides are present jointly in the proportion of 5 to 70 % of unsaturated triglycerides with 18 carbon atoms with three double bonds and 95 to 30 % of triglycerides with 16 carbon atoms, of which at least one third are unsaturated with a double bond.

2. A lipidic composition as claimed in claim 1, characterised in that more than 99 % of it is made up of a mixture of triglycerides of which more that 97 % are derivatives of fatty acids having between 14 and 18 carbon atoms.

3. A lipidic composition as claimed in claim 2, characterised in that it comprises, at the most, 1 % water or nonsaponifiable matter with more than 95 % cholesterol.

4. A lipidic composition as claimed in one of claims 1 to 3, characterised in that it has a viscosity of at the most 8 pascal/second at 20°C.

5. A lipidic composition as claimed in one of claims 1 to 4, characterised in that at least part of the unsaturated triglycerides of fatty acid with 16 carbon atoms come from extracts of at least one of mink or cow's foot oils.

6. A lipidic composition as claimed in on of claims 1 to 5, characterised in that at least part of the triglycerides of fatty acid with 18 carbon atoms and triple unsaturation come from extracts of at least one of soya, colza, walnut and raspberry seed oils.

7. A lipidic composition, as claimed in one of claims 5 and 6 characterised in that it is made up, at least partially, of extracts of animal and/or vegetable fatty substances.

8. A lipidic composition as claimed in claims 5 to 7, characterised in that at least part of the triglycerides of fatty acids with I6 or 18 carbons atoms from a purified mixture of 85 % mink oil and 15 % raspberry seed oil.

9. A cosmetic product, in particular shampoo, hair lotion, nail cream, nail varnish, nail varnish remover, characterised in that it comprises between 1 and 40 % of a lipidic composition as claimed in one of claims 1 to 8.